# EUROPEAN PATENT SPECIFICATION

(11) **EP 3 593 787 B1**
(45) Date of publication and mention of the grant of the patent: **13.10.2021**
(21) Application number: 19192950.4
(22) Date of filing: 24.07.2013
(51) Int. Cl.: A61K 8/34, A61K 8/41, A61Q 5/12, A61K 8/42, A61K 8/04

(54) **COMPOSITION**
ZUSAMMENSETZUNG
COMPOSITION

(30) Priority: 27.07.2012 EP 12178171; 27.07.2012 EP 12178168; 27.07.2012 EP 12178167; 27.07.2012 EP 12178165; 03.08.2012 EP 12179303
(43) Date of publication of application: 15.01.2020
(62) Divisional of application: 13740022.2
(73) Proprietor: Unilever IP Holdings B.V., 3013 AL Rotterdam (NL); Unilever Global IP Limited, Wirral, CH62 AZD (GB)
(72) Inventor: CASUGBO, Christia, Bebington, Wirral, Merseyside CH63 3JW (GB); FLANAGAN, Mark, Bebington, Wirral, Merseyside CH63 3JW (GB); HOUGH, John, Alan, Bebington, Wirral, Merseyside CH63 3JW (GB); NAUGHTON, John, Michael, Bebington, Wirral, Merseyside CH63 3JW (GB); SERRIDGE, David, Bebington, Wirral, Merseyside CH63 3JW (GB)
(74) Representative: Chisem, Janet

(56) References cited:
- EP-A1- 2 460 508

## Description

The present invention relates to a conditioning composition comprising superior conditioning capability.

EP-A-2460508 describes a hair conditioning composition comprising a cationic surfactant, a high melting point fatty compound and an agueous carrier, wherein the cationic surfactant, the high melting point fatty compound and the agueous carrier form a gel matrix.

Despite the prior art there remains a need for improved conditioning compositions.

Accordingly, the present invention provides a conditioning composition according to claim 1.

Draw Mass is the mass required to draw a control hair switch through a comb or brush. Thus the more tangled the hair the greater the mass required to pull the switch through the comb or brush.

The conditioning composition of the invention is obtainable by first forming a conditioning gel phase by:
forming a 'comelt' in a first vessel comprising fatty alcohol and cationic component and 0-15% wt. comelt water independently adding the 'comelt' and water to a mixing vessel mixing, wherein the temperature of the mixture of the 'comelt' and the water is maintained at from 56-65°C, preferably from 58-62°C, more preferably 60°C when in the mixing vessel, wherein the fatty alcohol comprises from 8 to 22 carbons, wherein the cationic component comprises from 0-70% cationic component, cationic surfactants have the formula N⁺R¹R²R³R⁴, more preferably from 30-60% wt. cationic surfactant component, and wherein R¹, R², R³ and R⁴ are independently (C₁ to C₃₀) alkyl or benzyl.

Conditioning compositions made using the conditioning gel phase of the invention are superior conditioning products. Specifically, they are thicker, despite having lower solids levels, and they are rinsed more easily. Products which are rinsed more easily use less water and so provide for a more sustainable future. These products are considered desirable by the environmentally aware consumer.

Preferably, the process is a continuous process.

The comelt of the invention forms an isotropic phase which means the development of structure, i.e. the formation of the lamellar conditioning gel phase,
can be controlled. In this process the temperature of the mixture of comelt and water is controlled by modifying the temperature of water added to the mix. Water may be added in one go or it may be staged. Typically, a first water vessel is maintained at around 40°C and is pumped into the mixing vessel while a second water vessel is maintained at a sufficient temperature to modify the temperature of the mixture of water with comelt such that it falls within the required range, i.e. from 56-65°C, preferably from 58-62°C, more preferably 60°C in the mixing vessel.

The conditioning composition ultimately made using such conditioning gel phase exhibits improved conditioning characteristics which are not observed when the conditioning gel phase is formed in the comelt.

The improvement thus resides in the balance of thermal energy at the point of mixing the water with the comelt.

If too cold then one ends up with a poorly mixed system due to the tendency for the comelt to solidify and this ultimately provides a composition of low viscosity. If the temperature of the mix vesicles form. This also gives rise to lower viscosity in the conditioning composition formed in the long run.

Preferably, the comelt comprises from 45-90% wt. comelt fatty alcohol.

Preferably, the fatty alcohol comprises from 8 to 22 carbon atoms, more preferably 16 to 22. Fatty alcohols are typically compounds containing straight chain alkyl groups. Examples of suitable fatty alcohols include cetyl alcohol, stearyl alcohol and mixtures thereof. The use of these materials is particularly preferable.

The level of fatty alcohol in the conditioner of the invention (not just the conditioning gel phase) will generally range from 0.01 to 10%, preferably from 0.1% to 8%, more preferably from 0.2 % to 7 %, most preferably from 0.3 % to 6 % by weight of the composition. The weight ratio of cationic surfactant to fatty alcohol is suitably from 1:1 to 1:10, preferably from 1:1.5 to 1:8, optimally from 1:2 to 1:5. If the weight ratio of cationic surfactant to fatty alcohol is too high, this can lead to eye irritancy from the composition. If it is too low, it can make the hair feel squeaky for some consumers.

Preferably, the comelt comprises from 10-40% wt. of the comelt cationic surfactant.

By adding cationic surfactants to fatty alcohol and stir at 85°C.

Inject this mixture into a flowing stream of water, containing other ingredients, the temperature of the water varied to ensure this mixture has a temperature of 60°C and mix.

Cool this stream towards ambient by injection into a second water stream and mix.

Suitable conditioning surfactants include those selected from cationic surfactants, used singly or in admixture. Preferably, the cationic surfactants have the formula N⁺R¹R²R³R⁴ wherein R¹, R², R³ and R⁴ are independently (C₁ to C₃₀) alkyl or benzyl. Preferably, one, two or three of R¹, R², R³ and R⁴ are independently (C4 to C₃₀) alkyl and the other R¹, R², R³ and R⁴ group or groups are (C₁-C₆) alkyl or benzyl. More preferably, one or two of R¹, R², R³ and R⁴ are independently (C₆ to C₃₀) alkyl and the other R¹, R², R³ and R⁴ groups are (C₁-C₆) alkyl or benzyl groups. Optionally, the alkyl groups may comprise one or more ester (-OCO- or -COO-) and/or ether (-O-) linkages within the alkyl chain. Alkyl groups may optionally be substituted with one or more hydroxyl groups. Alkyl groups may be straight chain or branched and, for alkyl groups having 3 or more carbon atoms, cyclic. The alkyl groups may be saturated or may contain one or more carbon-carbon double bonds (eg, oleyl). Alkyl groups are optionally ethoxylated on the alkyl chain with one or more ethyleneoxy groups.

Suitable cationic surfactants for use in the invention include cetyltrimethylammonium chloride, behenyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, dihydrogenated tallow dimethyl ammonium chloride (eg, Arquad 2HT/75 from Akzo Nobel), cocotrimethylammonium chloride, PEG-2-oleammonium chloride and the corresponding hydroxides thereof. Further suitable cationic surfactants include those materials having the CTFA designations Quaternium-5, Quaternium-31 and Quaternium-18. Mixtures of any of the foregoing materials may also be suitable. A particularly useful cationic surfactant for use in conditioners according to the invention is cetyltrimethylammonium chloride, available commercially, for example as GENAMIN CTAC, ex Hoechst Celanese. Another particularly useful cationic surfactant for use in conditioners according to the invention is behenyltrimethylammonium chloride, available commercially, for example as GENAMIN KDMP, ex Clariant.

Preferably, the cationic surfactant component of the comelt comprises from 0-70% cationic component, cationic surfactants have the formula N⁺R¹R²R³R⁴ as described above, more preferably from 30-60% wt. cationic surfactant component.

Another example of a class of suitable cationic surfactants for use in the invention, either alone or together with one or more other cationic surfactants, is a combination of (i) and (ii) below:
(i) an amidoamine corresponding to the general formula (I):

   (I) R1CONH(CH2)mN(R2)R3

   in which R¹ is a hydrocarbyl chain having 10 or more carbon atoms, R² and R³ are independently selected from hydrocarbyl chains of from 1 to 10 carbon atoms, and m is an integer from 1 to about 10; and
(ii) an acid.

As used herein, the term hydrocarbyl chain means an alkyl or alkenyl chain.

Preferred amidoamine compounds are those corresponding to formula (I) in which
R¹ is a hydrocarbyl residue having from about 11 to about 24 carbon atoms,
R² and R³ are each independently hydrocarbyl residues, preferably alkyl groups, having from 1 to about 4 carbon atoms, and m is an integer from 1 to about 4.

Preferably, R² and R³ are methyl or ethyl groups.

Preferably, m is 2 or 3, i.e. an ethylene or propylene group.

Preferred amidoamines useful herein include stearamido-propyldimethylamine, stearamidopropyldiethylamine, stearamidoethyldiethylamine, stearamidoethyldimethylamine, palmitamidopropyldimethylamine, palmitamidopropyldiethylamine, palmitamidoethyldiethylamine, palmitamidoethyldimethylamine, behenamidopropyldimethyl-amine, behenamidopropyldiethylmine, behenamidoethyldiethyl-amine, behenamidoethyldimethylamine, arachidamidopropyldimethylamine, arachidamidopropyldiethylamine, arachid-amidoethyldiethylamine, arachidamidoethyldimethylamine, and mixtures thereof.

Particularly preferred amidoamines useful herein are stearamidopropyldimethylamine, stearamidoethyldiethylamine, and mixtures thereof.

Commercially available amidoamines useful herein include: stearamidopropyldimethylamine with tradenames LEXAMINE S-13 available from Inolex (Philadelphia Pennsylvania, USA) and AMIDOAMINE MSP available from Nikko (Tokyo, Japan), stearamidoethyldiethylamine with a tradename AMIDOAMINE S available from Nikko, behenamidopropyldimethylamine with a
tradename INCROMINE BB available from Croda (North Humberside, England), and various amidoamines with tradenames SCHERCODINE series available from Scher (Clifton New Jersey, USA).

Acid may be any organic or mineral acid which is capable of protonating the amidoamine in the conditioner composition. Suitable acids useful herein include hydrochloric acid, acetic acid, tartaric acid, fumaric acid, lactic acid, malic acid, succinic acid, and mixtures thereof. Preferably, the acid is selected from the group consisting of acetic acid, tartaric acid, hydrochloric acid, fumaric acid, lactic acid and mixtures thereof.

The primary role of the acid is to protonate the amidoamine in the hair treatment composition thus forming a tertiary amine salt (TAS) in situ in the hair treatment composition. The TAS in effect is a non-permanent quaternary ammonium or pseudo-quaternary ammonium cationic surfactant.

Suitably, the acid is included in a sufficient amount to protonate more than 95 mole% (293 K) of the amidoamine present.

Should an amidoamine of the type described herein be present then the corresponding acid component will not be present in the comelt. Instead it will be
present in the water. Preferably, the water comprises protonating component at from 0.01 to 3% wt.

Accordingly, where the invention requires from 10-40% wt. comelt cationic surfactant, the cationic surfactant component may comprise amidoamine which is not protonated, i.e. it will not be cationic charged but will become protonated when added to the water and hence the protonating material contained therein.

Preferably, the cationic surfactant component of the comelt comprises from 0-70% cationic component, amidoamine corresponding to formula (I), more preferably from 30-60% wt. cationic surfactant component.

In conditioning compositions of the invention (not merely the conditioning gel phase), the level of cationic surfactant will generally range from 0.01 % to 10%, more preferably 0.05 % to 7.5%, most preferably 0.1 % to 5% by weight of the composition.

Preferably, where a comelt is used, the comelt is maintained at a melting point sufficient to maintain the fatty alcohol in a liquid phase. Preferably, the comelt is maintained at from
80-85C.

Preferably, the temperature of the mixture of the comelt and the water is controlled such that it is maintained from 56-65C, prefer from 58-62C, more preferably 60C during mixing.

Preferably, the contents of the mixture vessel passed through a mixer with rotor tip speed of 10-34, preferably from 21-27 and especially preferably 24 ms-1.

In a further aspect there is provided a process for manufacturing a conditioning composition by forming a conditioning gel phase obtained as described above and then adding any remaining ingredients. Typical remaining ingredients include fragrances, silicones, fibre actives or other benefit agents.

Preferably, the conditioning composition is passed through a mixer with rotor tip speed of 10-34, preferably from 21-27 and especially preferably 24 ms-1 one more time after the remaining ingredients have been added.

Conditioning compositions of the invention or using conditioning gel phases of the invention also deposit silicone better than conventionally made conditioning compositions.

Accordingly, the compositions of the invention can contain, emulsified droplets of a silicone conditioning agent, for enhancing conditioning performance.

Suitable silicones include polydiorganosiloxanes, in particular polydimethylsiloxanes which have the CTFA designation dimethicone. Also suitable for use compositions of the invention (particularly shampoos and conditioners) are polydimethyl siloxanes having hydroxyl end groups, which have the CTFA designation dimethiconol. Also suitable for use in compositions of the invention are silicone gums having a slight degree of crosslinking, as are described for example in WO 96/31188.

The viscosity of the emulsified silicone itself (not the emulsion or the final hair conditioning composition) is typically at least 10,000 cst at 25°C the viscosity of the silicone itself is preferably at least 60,000 cst, most preferably at least 500,000 cst, ideally at least 1,000,000 cst. Preferably the viscosity does not exceed 10⁹ cst for ease of formulation.

Emulsified silicones for use in the shampoo compositions of the invention will typically have an average silicone droplet size in the composition of less than 30, preferably less than 20, more preferably less than 10 micron, ideally from 0.01 to 1 micron. Silicone emulsions having an average silicone droplet size of
0.15 micron are generally termed microemulsions.

Emulsified silicones for use in the conditioner compositions of the invention will typically have an size in the composition of less than 30, preferably less than 20, more preferably less than 15. Preferably the average silicone droplet is greater than 0.5 micron, more preferably greater than 1 micron, ideally from 2 to 8 micron.

Silicone particle size may be measured by means of a laser light scattering technique, for example using a 2600D Particle Sizer from Malvern Instruments.

Examples of suitable pre-formed emulsions include Xiameter MEM 1785 and microemulsion DC2-1865 available from Dow Corning. These are emulsions /microemulsions of dimethiconol. Cross-linked silicone gums are also available in a pre-emulsified form, which is advantageous for ease of formulation.

A further preferred class of silicones for inclusion in shampoos and conditioners of the invention are amino functional silicones. By "amino functional silicone" is meant a silicone containing at least one primary, secondary or tertiary amine group, or a quaternary ammonium group. Examples of suitable amino functional silicones include: polysiloxanes having the CTFA designation "amodimethicone".

Specific examples of amino functional silicones suitable for use in the invention are the aminosilicone oils DC2-8220, DC2-8166 and DC2-8566 (all ex Dow Corning).

Suitable quaternary silicone polymers are described in EP-A-0 530 974. A preferred quaternary silicone polymer is K3474, ex Goldschmidt.

Also suitable are emulsions of amino functional silicone oils with non ionic and/or cationic surfactant.

Pre-formed emulsions of amino functional silicone are also available from suppliers of silicone oils such as Dow Corning and General Electric. Specific examples include DC939 Cationic Emulsion and the non-ionic emulsions DC2-7224, DC2-8467, DC2-8177 and DC2-8154 (all ex Dow Corning).

The total amount of silicone is preferably from 0.01 wt% to 10 %wt of the total composition more preferably from 0.1 wt% to 5 wt%, most preferably 0.5 wt% to 3 wt% is a suitable level.

### EXAMPLE 1

5g 10 inch (30cm) Virgin (not chemically damaged) Indian hair switches [industry standard hair type ex. International Hair Importers and Products, Glendale, NY] were base washed using 14% SLES, according to the base washing protocol.

### Base washing protocol

All switch washing to be done using the flow/temperature controlled taps. The flow rate is set at 4 litres / minute and a temperature of 35°C - 40°C.
1. Prep all of the syringes prior to starting to wash.
▪ Pre-fill the syringe with the base wash and empty
▪ Place the syringe on the balance and tare the balance
▪ Fill the syringe to the required mark and check on the balance that the correct amount for the two washes has been weighed out
▪ Repeat for each switch

2. Turn on tap and leave to stabilise for 30 seconds. The temperature and flow control is used by turning on the hot tap fully. Once the tap is turned on it is advisable to leave it running until all of the switches being treated in the session are done.
3. Wet out the switch by running it under the tap, remove excess water by running the first and middle finger down the length of the switch.
4. Lay the switch down flat on the edge of the sink and apply half of the measured Base Wash* (0.1ml/g hair) evenly down the length of the switch.
5. Holding both ends of the switch. Gently massage the base wash into the hair for 30 seconds. *Make sure to keep hold of both ends of the switch to avoid overly tangling the fibres.*
6. Rinse for 30 seconds, running the fingers down the switch every 5 seconds. Remove excess water.
7. Apply the remainder of the Base Wash evenly down the length of the switch.
8. Gently massage the Base Wash into the hair for 30 seconds, again holding both ends of the switch to avoid excess tangling.
9. Rinse for 30 seconds, running the fingers down the switch every 10 seconds. Remove excess water.
10. Lay the switch down on the edge of the sink and using the WIDE teeth of a Matador Sawcut No 4 comb; carefully comb the tangles out of the switch. *Comb down the switch from the root to the tip, starting at the tip and in sections work up slowly to the root.* Once all the tangles have been combed out finish with the NARROW teeth of the comb.
11. Run the first and middle finger down the switch and either dry at 50°C in the Drying Cabinet for a minimum of 2 hrs. Alternatively dry overnight at 20°C / 50% Relative Humidity.

| **Ingredient** | **Active(%)** | **INCI Name** | **Material Name** | **Formulation(%)** |
|---|---|---|---|---|
| Primary Surfactant | 70 | Texapon N701 | SLES-1 EO | 14.00 |
| Water | 100 | Aqua | Water | To 100% |
| | | | pH range 5.5 - 6.5 | |

A 5g 10" hair switch has approx 7000 fibres.

The switches were then dried in 50°C drying cabinet for two hours.

### Test protocol

The switches were then washed with the standard shampoo control formulation (see Table 1). The wash consisted of massaging in 0.1g shampoo per 1g of hair, for 30 seconds, followed by a 30 second rinse (water flow rate 4 I/min), then repeating these two steps.

The switches were then tested for detangling benefit using various conditioner test formulations.

The conditioner was used at a concentration of 0.2g of hair conditioner per 1g of hair. This was massaged into the switch for 1 minute and then rinsed for 5 seconds (water flow rate 4 l/min). The wet switch was placed onto a brush with a bulldog clip fastened to the glued end of the switch. The switch was placed on the brush such that from 5cm to 20cm was left hanging at the glued end.

Weights were added to the switch until the switch fell through the brush.

**Table 1**

| | |
|---|---|
| Table 1 presents the shampoo control for assessing Detangling Draw. The shampoo is made by standard processes. | |

| **INCI Name (CTFA)** | **%(W/W)** |
|---|---|
| Sodium Laureth Sulfate | 18.571 |
| Dimethiconol and Trideceth-10 and TEA-Dodecylbenzenesulfonate | 5.240 |
| Cocamidopropyl Betaine | 3.000 |
| Perfume | 0.750 |
| Ethylene Glycol Distearate/Sodium Laureth Sulphate/Cocomonoethanol amide | 9.302 |
| Glycerin | 0.500 |
| Acrylates/Beheneth-25 Methacrylate Copolymer | 1.000 |
| Amodimethicone and cetrimonium chloride and trideceth-12 | 1.140 |
| Guar Hydroxypropyltrimonium Chloride | 0.225 |
| Mica and Titanium Dioxide | 0.150 |
| Acrylates/Styrene Copolymer | 0.500 |
| Gluconolactone | 0.100 |
| Trehalose | 0.100 |
| Adipic Acid | 0.100 |
| Sodium Sulfate | 0.100 |
| Disodium EDTA | 0.100 |
| Guar Hydroxypropyltrimonium Chloride | 0.075 |
| PEG-45M | 0.075 |
| Preservative | 0.100 |
| Helianthus Annuus (Sunflower) Seed Oil | 0.010 |
| Preservative | 0.060 |
| Sodium Hydroxide | 0.150 |
| Citric Acid Monohydrate | 0.15 |
| Water | up to 100 |

### Conditioner compositions:

The comparative formulation was made by standard processes. The inventive formulation was made by processes as described above.

| | **Comparative** | **Inventive** |
|---|---|---|
| **INCI Name (CTFA)** | **%(W/W)** | **%(W/W)** |
| Cetearyl Alcohol | 4.000 | 3.1500 |
| Dimethicone and Amodimethicone and PEG-7 Propylheptyl Ether and Cetrimonium Chloride | 4.290 | 4.2900 |
| Behentrimonium Chloride | 1.630 | 1.3700 |
| Glycerin | 1.000 | 1.0000 |
| Perfume | 0.500 | 0.5000 |
| Stearamidopropyl Dimethylamine | 0.375 | 0.3200 |
| Lactic Acid | 0.120 | 0.1000 |
| Disodium EDTA | 0.100 | 0.1000 |
| Preservative | 0.100 | 0.100 |
| Sunflower Seed Oil | 0.010 | 0.0100 |
| Arginine HCL | 0.010 | 0.0100 |
| Lysine HCl | 0.010 | 0.0100 |
| Preservative | 0.060 | 0.0600 |
| Dye | 0.00013 | 0.00013 |
| Dye | 0.00013 | 0.00013 |
| Ammonium Hydroxide | 0.02000 | 0.02000 |
| Chlorinated water | up to 100 | up to 100.00 |

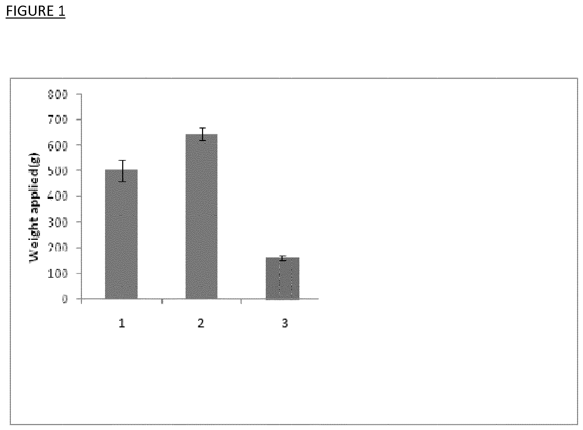

Figure 1 shows the weight required to draw switches.
1. Represents a switch washed with the control shampoo and then a product made according to the prior art methods (comparative product) where the conditioning gel phase is manufactured using standard processes.
2. Represents a switch washed with the control shampoo only.
3. Represents a switch washed with the control shampoo and then a formulation according to the invention which comprised lower levels of conditioning actives compared to the formulation in 1.

It can be seen that the mass required to pull the hair switch through the comb is greater for the comparative formulation than for the inventive formulation.

## Claims

1. Conditioning composition comprising from 0.4 to 8% wt. fatty alcohol having from 8-22 carbons, from 0.1 to 2% wt. cationic surfactant component, water, and wherein the composition confers a Draw Mass of from 1 to 250g to hair treated with the conditioning composition, comprising a conditioning gel phase obtainable by:
forming a 'comelt' in a first vessel comprising fatty alcohol and cationic component and 0-15% wt. comelt water, independently adding the 'comelt' and water to a mixing vessel, mixing,
wherein the temperature of the mixture of the 'comelt' and the water is maintained at from 56-65°C, preferably from 58-62°C, more preferably 60°C when in the mixing vessel, wherein the fatty alcohol comprises from 8 to 22 carbons, wherein the cationic component comprises from 0-70% by weight of cationic surfactant, cationic surfactants have the formula N⁺R¹R²R³R⁴, more preferably from 30-60% wt. cationic surfactant component, and wherein R¹, R², R³ and R⁴ are independently (C₁ to C₃₀) alkyl or benzyl,
and wherein the Draw Mass is the mass required to draw a hair switch through a comb or brush as measured by first placing the hair switch onto the comb or brush, such that from 5 cm to 20 cm of hair is left hanging at the glued end of the switch, and then adding weights to the hanging end until the switch falls through the comb or brush.

2. A composition according to Claim 1 wherein the process is a continuous process.

3. A composition according to Claim 2 wherein the comelt forms an isotropic phase.

4. A composition according to any of the preceding claims wherein the addition of the water is added in one go or staged.

5. A composition according to any of the preceding claims wherein a first water vessel is maintained at around 40°C and is pumped into the mixing vessel while a second water vessel is maintained at a sufficient temperature to modify the temperature of the mixture of water with comelt such that it falls within the required range.

6. A composition according to any of the preceding claims wherein the comelt comprises 45-90% by weight of the comelt of fatty alcohol.

7. A composition according to any of the preceding claims wherein the comelt comprises 10-40% by weight of the comelt of cationic surfactant.

8. A composition according to any of the preceding claims where the level of fatty alcohol in the conditioning composition is from 0.01 to 10%, most preferably from 0.3% to 6% by weight of the composition.

9. A composition according to any of the preceding claims wherein the weight ratio of cationic surfactant is suitably from 1:1 to 1:10, optimally 1:2 to 1:5.

## Patentansprüche

1. Konditionierungszusammensetzung, umfassend 0,4 bis 8 Gew.-% Fettalkohol mit 8-22 Kohlenstoffen, 0,1 bis 2 Gew.-% kationische Tensidkomponente, Wasser, und wobei die Zusammensetzung Haar, das mit der Konditionierungszusammensetzung behandelt ist, eine Ziehmasse von 1 bis 250 g verleiht, und eine konditionierende Gelphase umfasst, erhältlich durch:
Bilden einer "Coschmelze" in einem ersten Behälter umfassend Fettalkohol und kationische Komponente und 0-15 Gew.-% der Coschmelze an Wasser, wobei die "Coschmelze" und Wasser unabhängig in einen Mischbehälter gegeben werden, Mischen,
wobei die Temperatur der Mischung von der "Coschmelze" und dem Wasser bei 56-65 °C, bevorzugt 58-62 °C, bevorzugter 60 °C, gehalten wird, wenn sie im Mischbehälter ist, wobei der Fettalkohol 8 bis 22 Kohlenstoffe umfasst, wobei die kationische Komponente 0-70%, bezogen auf das Gewicht des kationischen Tensids, wobei kationische Tenside die Formel N⁺R¹R²R³R⁴ aufweisen, bevorzugter 30-60 Gew.% kationische Tensidkomponente, umfasst, und wobei R¹, R², R³ und R⁴ unabhängig (C₁ bis C₃₀) Alkyl oder Benzyl sind,
und wobei die Ziehmasse die Masse ist, die erforderlich ist, um ein Haarteil durch einen Kamm oder eine Bürste zu ziehen, gemessen, indem zuerst das Haarteil auf den Kamm oder die Bürste platziert wird, so dass 5 cm bis 20 cm Haar hängend verbleiben am verklebten Ende des Haarteils, und dann Gewichte am hängenden Ende hinzugefügt werden, bis das Haarteil durch den Kamm oder die Bürste fällt.

2. Zusammensetzung nach Anspruch 1, wobei das Verfahren ein kontinuierliches Verfahren ist.

3. Zusammensetzung nach Anspruch 2, wobei die Coschmelze eine isotrope Phase bildet.

4. Zusammensetzung nach irgendeinem der vorhergehenden Ansprüche, wobei die Zugabe des Wassers auf einmal oder stufenweise erfolgt.

5. Zusammensetzung nach irgendeinem der vorhergehenden Ansprüche, wobei ein erster Wasserbehälter bei etwa 40 °C gehalten und in den Mischbehälter gepumpt wird, während ein zweiter Wasserbehälter bei einer ausreichenden Temperatur gehalten wird, um die Temperatur der Mischung von Wasser mit Coschmelze zu modifizieren, so dass sie in den erforderlichen Bereich fällt.

6. Zusammensetzung nach irgendeinem der vorhergehenden Ansprüche, wobei die Coschmelze 45-90%, bezogen auf das Gewicht der Coschmelze, Fettalkohol umfasst.

7. Zusammensetzung nach irgendeinem der vorhergehenden Ansprüche, wobei die Coschmelze 10-40%, bezogen auf das Gewicht der Coschmelze, kationisches Tensid umfasst.

8. Zusammensetzung nach irgendeinem der vorhergehenden Ansprüche, wobei der Gehalt an Fettalkohol in der Konditionierungszusammensetzung 0,01 bis 10%, am meisten bevorzugt 0,3 bis 6%, bezogen auf das Gewicht der Zusammensetzung, beträgt.

9. Zusammensetzung nach irgendeinem der vorhergehenden Ansprüche, wobei das Gewichtsverhältnis von kationischem Tensid geeigneterweise 1:1 bis 1:10, optimal 1:2 bis 1:5, beträgt.

## Revendications

1. Composition de conditionnement comprenant de 0,4 à 8 % en masse d'alcool gras ayant 8-22 atomes de carbone, de 0,1 à 2 % en masse de constituant de tensioactif cationique, de l'eau, et dans laquelle la composition confère une masse d'étirage de 1 à 250 g aux cheveux traités avec la composition de conditionnement, comprenant une phase de gel de conditionnement pouvant être obtenue par :
formation d'une 'cofusion' dans une première cuve comprenant un alcool gras et un constituant cationique et 0-15 % en masse d'eau de cofusion, addition indépendante de la 'cofusion' et eau à une cuve de mélange, mélange,
dans laquelle la température du mélange de la 'cofusion' et de l'eau est maintenue à 56-65°C, de préférence 58-62°C, encore mieux 60°C lorsqu'il se trouve dans la cuve de mélange, dans laquelle l'alcool gras comprend de 8 à 22 atomes de carbone, dans laquelle le constituant cationique comprend 0-70 % en masse de tensioactif cationique, les tensioactifs cationiques présentent la formule N⁺R¹R²R³R⁴, encore mieux 30-60 % en masse de constituant de tensioactif cationique, et dans laquelle R¹, R², R³ et R⁴ sont indépendamment un groupe alkyle en (C₁ à C₃₀) ou benzyle,
et dans laquelle la masse d'étirage est la masse nécessaire pour tirer une tresse de cheveux à travers un peigne ou une brosse comme mesurée en plaçant dans un premier temps la tresse de cheveux sur le peigne ou la brosse, de sorte que de 5 cm à 20 cm de cheveux sont laissés suspendus à l'extrémité collée de la tresse, et en ajoutant ensuite des masses à l'extrémité suspendue jusqu'à ce que la tresse tombe à travers le peigne ou la brosse.

2. Composition selon la revendication 1, dans laquelle le procédé est un procédé continu.

3. Composition selon la revendication 2, dans laquelle la cofusion forme une phase isotrope.

4. Composition selon l'une quelconque des revendications précédentes, dans laquelle l'addition de l'eau est ajoutée en une fois ou en étapes.

5. Composition selon l'une quelconque des revendications précédentes, dans laquelle une première cuve d'eau est maintenue à environ 40°C et est pompée dans la cuve de mélange alors qu'une seconde cuve d'eau est maintenue à une température suffisante pour modifier la température du mélange d'eau avec la cofusion de sorte qu'elle se trouve dans l'intervalle requis.

6. Composition selon l'une quelconque des revendications précédentes, dans laquelle la cofusion comprend 45-90 % en masse de la cofusion d'alcool gras.

7. Composition selon l'une quelconque des revendications précédentes, dans laquelle la cofusion comprend 10-40 % en masse de la cofusion de tensioactif cationique.

8. Composition selon l'une quelconque des revendications précédentes, dans laquelle la teneur en alcool gras dans la composition de conditionnement est de 0,01 à 10 %, encore mieux de 0,3 % à 6 % en masse de la composition.

9. Composition selon l'une quelconque des revendications précédentes, dans laquelle le rapport en masse de tensioactif cationique est de manière appropriée de 1:1 à 1:10, de manière optimale de 1:2 à 1:5.
